# EUROPEAN PATENT APPLICATION

(11) **EP 4 487 851 A1**
(43) Date of publication of application: **08.01.2025**
(21) Application number: 23763735.0
(22) Date of filing: 03.03.2023
(51) Int. Cl.: A61K 31/517, A61P 17/16, A23L 33/10, A61K 8/49, A61Q 19/00, A61Q 17/04

(54) **PHARMACEUTICAL COMPOSITION FOR PREVENTING OR TREATING SKIN DISEASES CAUSED BY RADIATION IRRADIATION COMPRISING DARAPLADIB OR PHARMACEUTICALLY ACCEPTABLE SALT THEREOF AS ACTIVE INGREDIENT**

(30) Priority: 04.03.2022 KR 20220028028
(71) Applicant: Korea Institute of Radiological & Medical Sciences, Seoul 01812 (KR)
(72) Inventor: KIM, Kwang Seok, Seoul 01747 (KR); KANG, Chang Mo, Seoul 01913 (KR); KIM, So Ra, Seoul 02803 (KR)
(74) Representative: Mewburn Ellis LLP
(86) International application number: PCT/KR2023/002922
(87) International publication number: WO 2023/167537

(57) **Abstract**

The present invention relates to a pharmaceutical composition for preventing or treating skin diseases caused by radiation irradiation, comprising darapladib or a pharmaceutically acceptable salt thereof as an active ingredient. Darapladib improves damaged skin due to radiation irradiation, such as maintaining the water loss amount, water content level and pH level of damaged skin due to radiation irradiation, and recovering skin thickness and adipocyte area, and thus can be effectively used as a composition for preventing, treating or ameliorating skin diseases caused by radiation irradiation.

## Description

### Technical Field

The present disclosure relates to a pharmaceutical composition for preventing or treating skin diseases caused by radiation irradiation, including darapladib or a pharmaceutically acceptable salt thereof as an active ingredient.

### Background Art

Radiotherapy has been used to treat approximately 50% of all cancer patients, contributing to a remarkable reduction in cancer mortality. The use of radiotherapy is an effective way to treat many types of cancer, but it may lead to some complications. Radiotherapy, which achieves most of its cell-killing properties by generating oxygen radicals within cells, may damage DNA of non-cancer cells, affecting growth of these normal cells, especially those with mitotic activity.

Although irradiation is utilized in a variety of ways to treat cancer, it may also damage healthy cells, causing acute or chronic side effects. In particular, radiodermatitis and radiation fibrosis, which frequently develop in skin or soft tissues after radiotherapy, not only occur after a long time through the abnormal wound healing process, but also worsen continuously, fatally degrading the quality of life. During the normal wound healing process, fibroblasts are activated into a form of myofibroblasts to induce cell proliferation, and after accumulation of collagen, this process is interrupted by normal immune activity and feedback. Unfortunately, in radiation-induced fibrosis, this process does not work properly, resulting in continuous activation of myofibroblasts, loss of function due to abnormal accumulation of collagen, and tissue fibrosis. Recent studies have reported that the reduction of adipocytes, including white adipose tissue, which is present in the dermis layer of the skin and regulates immune activity at the site of a lesion where skin fibrosis has developed, plays an important role. Radiation fibrosis, which occurs very frequently after radiotherapy, has limited treatment options in comparison with the frequency and severity, with minimal research on the mechanism thereof.

### Disclosure of the Invention

### Technical Goals

An object of the present disclosure is to provide a pharmaceutical composition for preventing or treating skin diseases caused by radiation irradiation, including darapladib or a pharmaceutically acceptable salt thereof as an active ingredient.

Another object of the present disclosure is to provide a health functional food composition for preventing or ameliorating skin diseases caused by radiation irradiation, including darapladib or a foodologically acceptable salt thereof as an active ingredient.

Another object of the present disclosure is to provide a cosmetic composition for preventing or ameliorating skin diseases caused by radiation irradiation, including darapladib or a cosmetically acceptable salt thereof as an active ingredient.

### Technical Solutions

To achieve the above objectives, the present disclosure provides a pharmaceutical composition for preventing or treating skin diseases caused by radiation irradiation, including darapladib or a pharmaceutically acceptable salt thereof as an active ingredient.

In addition, the present disclosure provides a health functional food composition for preventing or ameliorating skin diseases caused by radiation irradiation, including darapladib or a foodologically acceptable salt thereof as an active ingredient.

In addition, the present disclosure provides a cosmetic composition for preventing or ameliorating skin diseases caused by radiation irradiation, including darapladib or a cosmetically acceptable salt thereof as an active ingredient.

### Advantageous Effects

According to the present disclosure, darapladib maintains the moisture loss, moisture content level, and pH level in the skin damaged by radiation irradiation and improves skin damaged by radiation irradiation, such as restoration of skin thickness and area of adipocytes, such that the darapladib may be useful as a composition for preventing, treating, or ameliorating skin diseases caused by radiation irradiation.

### Brief Description of Drawings

FIG. 1 shows a result of analyzing an effect of darapladib (Dara) on suppression of differentiation from fibroblasts to myofibroblasts.
FIG. 2 shows results of analyzing an excessive cell proliferation suppressive effect of darapladib (Dara) in skin fibroblasts.
FIG. 3 shows results of analyzing an irradiation-induced skin damage ameliorating effect of darapladib (Dara).
FIG. 4 shows results of analyzing changes in skin moisture content, moisture loss, and skin pH by irradiation using darapladib (Dara).
FIG. 5 shows results of analyzing histological changes in the skin by irradiation using darapladib (Dara). A represents a thickness of epidermis and dermis in the skin and granules of adipocytes, B represents an area of adipocytes, and C represents changes in the number of adipocytes.
FIG. 6 shows results of analyzing an effect of darapladib (Dara) on suppression of irradiation-induced collagen deposition and overexpression in skin.
FIG. 7 shows results of analyzing an effect of darapladib (Dara) on suppression of α-SMA expression.
FIG. 8 shows results of analyzing an effect of darapladib (Dara) on suppression of vimentin expression.

### Best Mode for Carrying Out the Invention

Hereinafter, the present disclosure will be described in more detail.

With efforts to develop a treatment for skin diseases caused by radiation irradiation, which is one of the side effects of radiotherapy, the present inventor has completed the present disclosure by determining that darapladib maintains an amount of moisture loss, moisture content level, and pH level of the skin damaged by irradiation and shows an effect of ameliorating skin damaged by irradiation, such as restoration of the skin thickness and adipocyte area.

The present disclosure provides a pharmaceutical composition for preventing or treating a skin disease caused by radiation irradiation, including darapladib or a pharmaceutically acceptable salt thereof as an active ingredient.

The darapladib or pharmaceutically acceptable salt thereof may inhibit expression of one or more selected from the group consisting of collagen, α-SMA, and vimentin, but is not limited thereto.

In addition, the darapladib or pharmaceutically acceptable salt thereof may suppress differentiation from fibroblasts to myofibroblasts; or overgrowth of fibroblasts.

The skin disease may include, but is not limited to, fibrosis or dermatitis.

The radiation irradiation may be high-dose irradiation, and the radiation may range from 10 to 40 Gy, but is not limited thereto.

The pharmaceutical composition of the present disclosure may be prepared in the form of a unit dose by formulation using a pharmaceutically acceptable carrier or prepared by encapsulating into a multi-capacity container, in accordance with a method that may be easily carried out by a person skilled in the art to which the disclosure pertains.

The pharmaceutically acceptable carriers are commonly used in preparation, including, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition of the present disclosure may further include lubricants, humectants, sweeteners, flavoring agents, emulsifiers, suspensions, and preservatives, in addition to the above ingredients.

In the present disclosure, the content of the additives included in the pharmaceutical composition is not particularly limited and may be appropriately adjusted within a content range used in the conventional preparation.

The pharmaceutical composition may be formulated in the form of one or more external skin agents selected from the group consisting of, but are not limited to, injectable formulations such as aqueous solutions, suspensions, and emulsions, pills, capsules, granules, tablets, creams, gels, patches, sprays, ointments, plasters, lotions, liniments, pastas, and cataplasmas.

The pharmaceutical composition of the present disclosure may include a pharmaceutically acceptable carrier and diluent that are additionally present for formulation. The pharmaceutically acceptable carriers and diluents include, but are not limited to, excipients such as starch, sugars, and mannitol, fillers and extenders such as calcium phosphate, cellulose derivatives such as carboxymethylcellulose and hydroxypropylcellulose, binders such as gelatin, alginate, and polyvinyl pyrrolidone, lubricants such as talc, calcium stearate, hydrogenated castor oil, and polyethylene glycol, disintegrating agents such as povidone and crospovidone, and surfactants such as polysorbate, cetyl alcohol, and glycerol. The pharmaceutically acceptable carriers and diluents may be biologically and physiologically friendly to a subject. Examples of diluents may include, but are not limited to, brine, water-soluble buffers, solvents, and/or dispersion media.

The pharmaceutical composition of the present disclosure may be administered orally or parenterally (e.g., applied intravenously, subcutaneously, intraperitoneally, or topically) depending on the desired method. When administered orally, it may be formulated as tablets, troches, lozenges, water-soluble suspensions, oil-based suspensions, powder preparation, granules, emulsions, hard capsules, soft capsules, syrups, and elixirs. When administered parenterally, it may be formulated as injection solutions, suppositories, powders for respiratory inhalation, aerosols for sprays, ointments, powders for application, oils, and creams.

The dosage of the pharmaceutical composition of the present disclosure may vary depending on the patient's condition, weight, age, sex, health status, dietary constitution specificity, nature of the preparation, severity of disease, administration time for composition, method of administration, duration or interval of administration, excretion rate, and drug form, and may be appropriately selected by a person skilled in the art. For example, it may range from about 0.1 to 10,000 mg/kg, but is not limited thereby, and it may be administered once or several times a day.

The pharmaceutical composition may be administered orally or parenterally (e.g., applied intravenously, subcutaneously, intraperitoneally, or topically) depending on the desired method. The pharmaceutically effective amount and effective dosage of the pharmaceutical composition of the present disclosure may vary by the preparation method of the pharmaceutical composition, type of administration, administration time, and administration route, and a person with ordinary skill in the art may easily determine and prescribe the effective dosage for the desired treatment. The administration of the pharmaceutical composition of the present disclosure may be administered once a day or in several divided doses.

In addition, the present disclosure provides a health functional food composition for preventing or ameliorating skin diseases caused by radiation irradiation, including darapladib or a foodologically acceptable salt thereof as an active ingredient.

The present disclosure may be used generally as a food product that is conventionally used.

The food composition of the present disclosure may be used as a health functional food. The term "health functional food" as used herein refers to a food manufactured and processed using raw materials or ingredients that have useful functionality for the human body in accordance with the Health Functional Food Act, and the term "functionality" as used herein refers to consumption for the purpose of deriving useful effects for health purposes such as adjusting nutrients or physiological actions on the structure and function of the human body.

The health functional food composition may include conventional food additives, and the suitability as the "food additive" is determined by the standards and criteria related to corresponding items according to the general rules and general test methods of Korean Food Additives Codex approved by the Ministry of Food and Drug Safety, unless otherwise stipulated.

The items listed in the "Korean Food Additives Codex" may include, for example, chemically synthesized compounds such as ketones, glycine, potassium citrate, nicotinic acid, and cinnamic acid, natural additives such as persimmon color, licorice extracts, crystallized cellulose, kaoliang color, and guar gum, and mixed preparations such as sodium L-glutamate preparations, noodle-added alkali agents, preservative agents, and tar color agents.

The food composition of the present disclosure may be manufactured and processed in the form of tablets, capsules, powder, granules, liquids, and pills. For example, hard capsules among health functional foods in the form of capsules may be prepared by mixing and filling the composition according to the present disclosure in conventional hard capsules along with additives such as excipients, and the soft capsules may be manufactured by mixing the composition according to the present disclosure with the additives such as excipients and then filling the same in capsule bases such as gelatin. The soft capsule may contain, if necessary, plasticizers such as glycerin or sorbitol, colorants, and preservatives.

The definition of terms for the excipient, binder, disintegrant, lubricant, flavor enhancer, and flavoring agent is described in documents known in the art and includes those having the same or similar functions. The type of food is not particularly limited and includes all health functional foods in the ordinary sense.

The term "prevention" as used herein refers to any action of suppressing or delaying diseases by administering the composition according to the present disclosure. The term "treatment" as used herein refers to any action that ameliorates or favorably changes the symptoms of diseases by administering the composition according to the present disclosure. The term "amelioration" as used herein refers to any action of making the bad condition of diseases better by having an individual administer or ingest the composition of the present disclosure.

In addition, the present disclosure provides a cosmetic composition for preventing or ameliorating skin diseases caused by radiation irradiation, including darapladib or a cosmetically acceptable salt thereof as an active ingredient.

The cosmetic composition has one or more formulations selected from the group consisting of, but is not limited to, skin lotions, skin softeners, skin toners, astringents, milk lotions, moisture lotions, nourishing lotions, massage creams, nourishing creams, moisture creams, hand creams, foundations, essences, nourishing essences, packs, soaps, cleansing foams, cleansing lotions, cleansing creams, body lotions, and body cleansers.

The term "including as an active ingredient" as used herein may refer to inclusion of an effective amount that may show the effect of ameliorating skin diseases.

Specifically, the cosmetic composition may be prepared in a general emulsifying formulation and a solubilized formulation. For example, it may be formulated into astringents such as softening astringents or nourishing astringents; emulsions such as facial lotions and body lotions; creams such as nourishing creams, moisturizing creams, and eye creams; essences; cosmetic ointments; sprays; gels; packs; sunscreens; makeup bases; foundations such as liquid type, solid type, or spray type; powders; makeup removers such as cleansing creams, cleansing lotions, and cleansing oils; or cleansers such as cleansing foams, soaps, and body washes, but is not limited to. In addition, the external skin agent may be formulated as an ointment, patch, gel, cream or spray, but is not limited to.

For each formulation, in addition to the essential ingredients, the cosmetic composition may be appropriately combined with other ingredients, within a range that it does not interfere with the purpose according to the present disclosure depending on the type of formulation or the purpose of use.

The cosmetic composition may include a conventionally acceptable carrier, for example, it may be appropriately combined with oil, water, surfactants, moisturizers, low-grade alcohol, thickeners, chelating agents, colors, preservatives, and fragrances, but is not limited to.

The acceptable carrier may vary depending on the formulation. For example, animal oils, vegetable oils, waxes, paraffins, starches, tracanths, cellulose derivatives, polyethylene glycol, silicones, bentonite, silica, talc, zinc oxide or extracts thereof may be used as carrier components when formulated into ointments, pastes, creams, or gels.

When the cosmetic composition is formulated as the powder or spray, lactose, talc, silica, aluminum hydroxide, calcium silicate, polyamide powder, or an extract thereof may be used as the carrier component, and in the case of the spray, a booster such as chlorofluorohydrocarbon, propane, butane or dimethyl ether may be additionally included.

When the cosmetic composition is formulated as the solution or emulsion, solvents, solubilizers, or emulsifiers may be used as the carrier component, for example water, ethanol, isopropanol, ethyl carbonate, ethyl acetate, benzyl benzoate, propylene glycol, and 1,3-bthyl glycol oil may be used, in particular, cottonseed oil, peanut oil, corn germ oil, olive oil, castor oil and sesame seed oil, glycerol aliphatic esters, polyethylene glycol, or fatty acid esters of sorbitan may be used.

When the cosmetic composition is formulated as the suspension, water, liquid diluents such as ethanol or propylene glycol, ethoxylated isostearyl alcohol, suspension agents such as polyoxyethylene sorbitol ester and polyoxyethylene sorbitan ester, microcrystalline cellulose, aluminum metahydroxide, bentonite, agar, or tracanth may be used as the carrier component.

When the cosmetic composition is formulated into soap, alkali metal salts of fatty acids, fatty acid hemiester salts, fatty acid protein hydrolyzate, isethionate, lanolin derivatives, aliphatic alcohol, vegetable oil, glycerol, and sugar may be used as the carrier components.

The cosmetic composition may additionally contain supplements that are commonly used in the field of cosmetology or dermatology, such as fatty substances, organic solvents, solubilizers, thickeners, gelling agents, emollients, antioxidants, suspensions, stabilizers, foaming agents, aromatic agent, surfactants, water, ionic or non-ionic emulsifiers, fillers, metal ion sequestering agent, chelating agents, preservatives, blockers, humectants, essential oils, dyes, pigments, hydrophilic or lipophilic activators, and any other ingredients commonly used in cosmetics, which are conventionally used in the art depending on the quality or function of final products.

However, the supplement and a mixing ratio thereof may be appropriately selected so as not to affect the desirable properties of the cosmetic composition according to the present disclosure.

### Modes for Carrying Out the Invention

Hereinafter, the present disclosure will be described in detail through examples to help understanding of the present disclosure. However, the following examples are intended to merely illustrate the content of the present disclosure, but the scope of the present disclosure is not limited to the following examples. Examples of the present disclosure are provided to more completely explain the present disclosure to those with ordinary skill in the art.

### [Example 1] Analysis of effect of darapladib on suppression of skin fibroblast growth and differentiation

To identify the effect of darapladib on suppression of differentiation from fibroblasts to myofibroblasts, used was a transforming growth factor-beta (TGF-β), a growth factor that induces differentiation from human fibroblasts to myofibroblasts. Darapladib was applied to the medium at low concentration (0.25 nM) and high concentration (0.5 nM) respectively 1 hour before TGF-β treatment and then fixed with 4% formaldehyde in a live state in a single-layer culture of fibroblasts, and immunofluorescence staining of collagen was performed, followed by visualization by confocal microscopy.

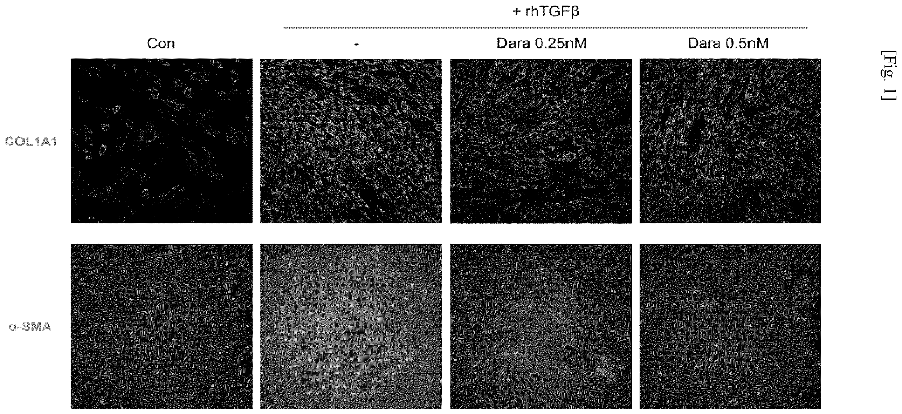

As a result, it was found that the expression of collagen increased in a TGF-β treated group, while that of collagen decreased significantly in a darapladib treated group (Dara), and in particular, the expression of collagen was restored in the high-concentration treated group (Dara 0.5nM) similar to that of a control group without irradiation (FIG. 1).

In addition, vascular endothelial growth factor (VEGF), another growth factor in addition to TGF-β, was treated every day for 4 days to induce the growth of fibroblasts and find out whether darapladib inhibits the excessive growth of fibroblasts.

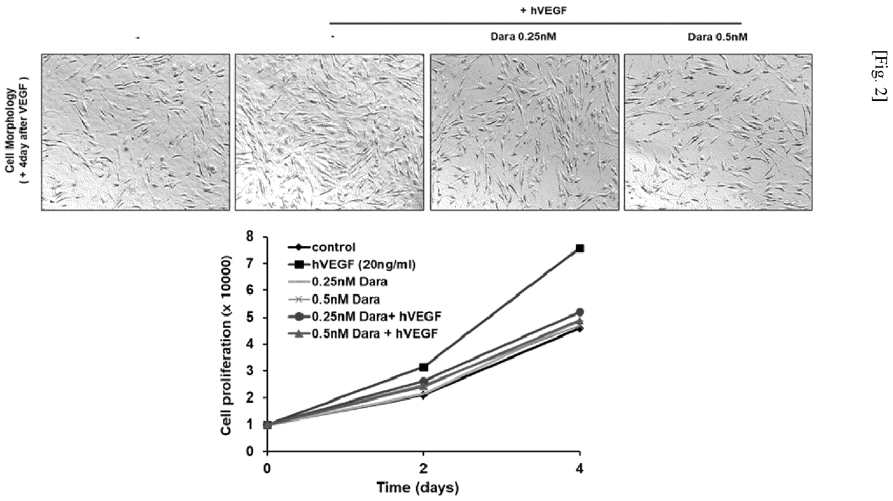

As a result, it was found that, on day 4, the number of fibroblasts increased significantly in the VEGF-treated group (hVEGF) compared to the control group, while the number of fibroblasts in the darapladib-treated group showed the similar number of fibroblasts to the control group (FIG. 2).

### [Example 2] Analysis of effect of darapladib in inhibiting radiation-induced skin damage

In order to identify the effect of darapladib in improving radiation-induced skin damage, SKH-1 hairless mice, which has the most similar characteristics to the human skin layer, immune system, and vascular system, was used, and for the irradiated group, local irradiation was performed on the skin in a total of 40 Gy by irradiating two times by 20 Gy at intervals of 5 days. Darapladib was used to suppress radiation-induced skin damage, and subcutaneous injections were given at different concentrations (20 mg/kg and 40 mg/kg).

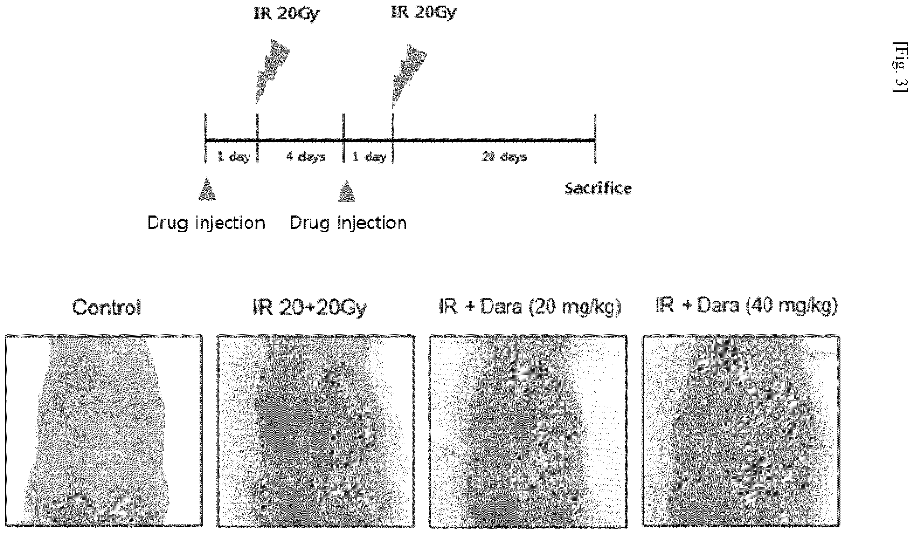

As a result, it was found that fibrosis with chronic thickening of skin surface, red wounds, or inflammation was observed in the irradiated group (IR) on day 20 after irradiation, while fibrosis was remarkably reduced in the darapladib treated group (IR+Dara), and particularly high-concentration treated group (40mg/kg) showed recovery similar to that of the control group without irradiation (FIG. 3).

In addition, in order to quantitatively observe the effect of darapladib in inhibiting radiation-induced skin damage, the moisture content, moisture loss, and pH level of the skin were measured on day 20 after irradiation. The moisture content in the skin was measured using Corneometer (Corneometer CM825, CK electronic GmbH, Germany), the moisture loss by Tewameter (TM300, CK electronic GmbH, Germany), and the pH level of the skin by a skin pH meter (pH905, CK electronic GmbH, Germany).

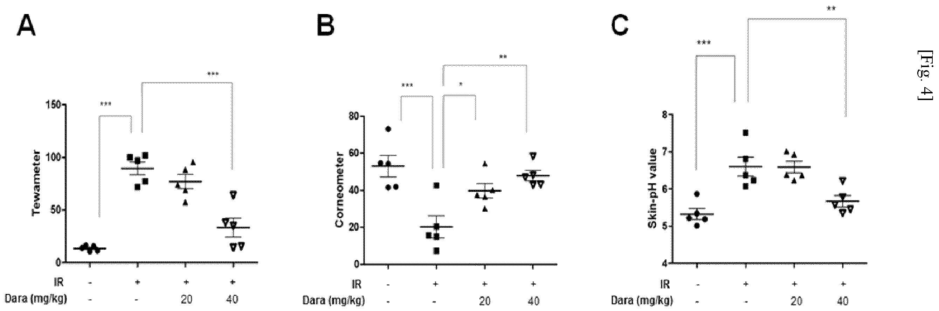

As a result, it was found that the irradiated group had significantly high transdermal moisture loss, low moisture content, and slightly basic skin pH compared to the control group, while the darapladib treated group maintained the moisture loss and moisture content level similar to that of the control group, with the skin pH remained slightly acidic as well (FIG. 4).

In addition, in order to observe the histological changes in the skin, the extracted tissues were embedded by undergoing a paraffin infiltration process, a 4µm thinned section was prepared to undergo deparaffinization and hydration followed by staining with hematoxylin and eosin, and then tissue damage, blood vessel damage, and changes in the epidermis, dermis, and adipocyte layer of skin tissue were observed using an optical microscope. In addition, to quantify the degree of skin damage, the area and number of granules in which adipocytes are present were calculated and statistically analyzed using the Image J program.

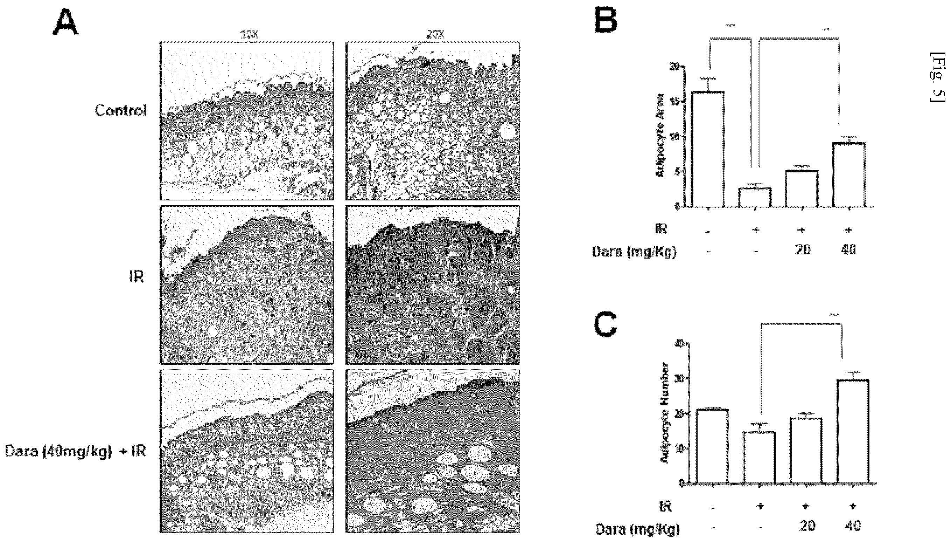

As a result, compared to the control group, the thickness of the epidermis and dermis considerably increased in the skin sections of the irradiated group (IR), and the collagen and extracellular matrix fibers in the skin and soft tissues showed an irregular arrangement. In addition, it was found that myofibroblasts increased, the formation of fibroblasts near the epidermis was prominent, and the number and area of adipocyte granules decreased. On the other hand, in the darapladib treated group (Dara+IR), it was found that the thickness of the epidermis and dermis of the skin was restored similar to the control group, and the adipocyte granules were retained (FIG. 5A). In addition, it was found that the area of adipocytes was significantly reduced by irradiation, but significantly recovered by darapladib treatment (FIGS. 5B and 5C).

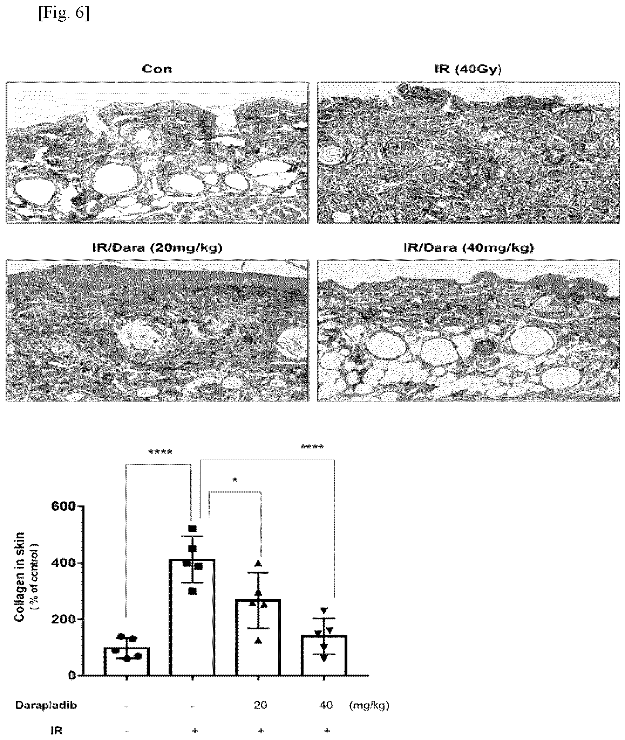

In addition, as a result of observing the skin layer by taking the skin on day 20 after irradiation and then subjecting to Masson's trichrome staining, it was determined that the irradiated group (IR) had collagen fibers chronically increased in the dermis layer of the skin and the thickened skin layer on day 20 after irradiation, while darapladib treated group (IR/Dara) had collagen fiber or the thickness of the skin layer restored to a level of the control group (Con) (FIG. 6).

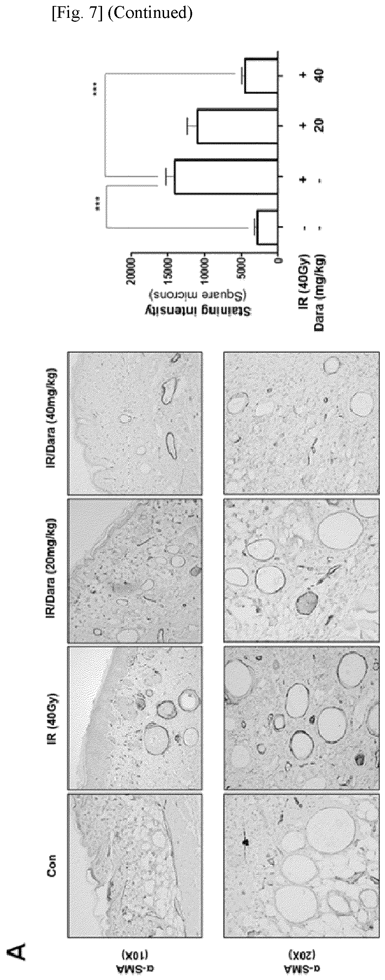

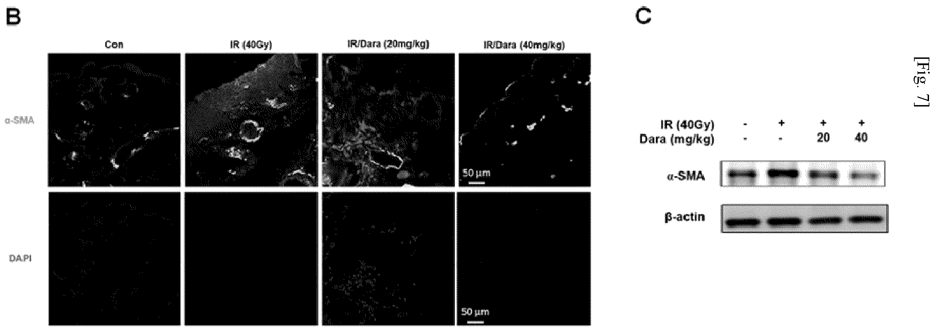

In addition, with the expression of α-SMA, a marker of myofibroblasts and a marker of fibrosis, as a result of identifying the expression level and the location thereof via IHC staining, immunofluorescence (IF), and Western blot, respectively, it was found that the irradiated group (IR) had increased expression of α-SMA in the epidermis, dermis layer, and adipocyte layer of the skin, whereas the darapladib treated group (IR/Dara) had reduced expression of α-SMA compared to the irradiated group, and in particular, the high-concentration treated group (40mg/kg) had the expression level similar to that of the control group (FIG. 7).

In addition, to identify the effect of darapladib on vimentin, which is another marker of fibrosis and applicable to immune diseases, immunohistochemistry (IHC) method was applied to stain the surface of the skin.

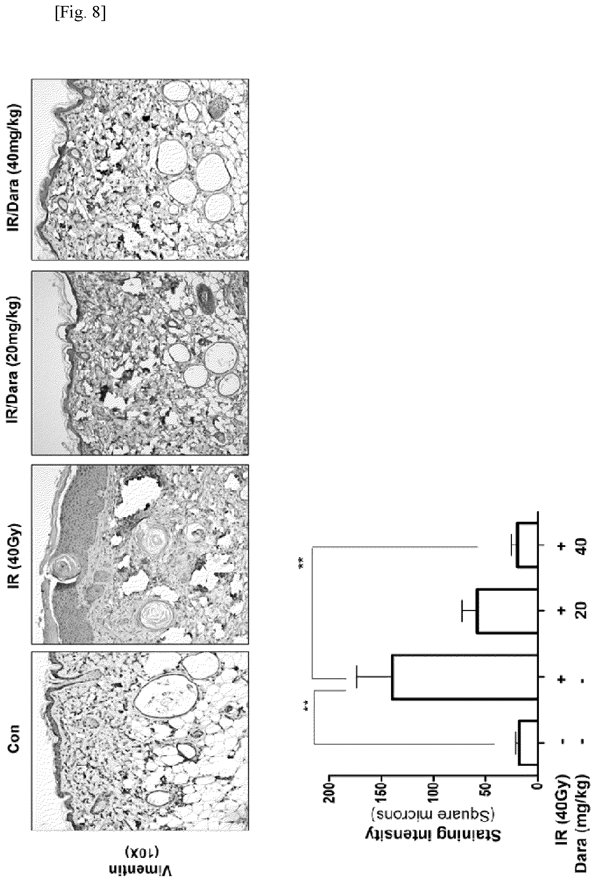

As a result, the expression of vimentin in the epidermis and dermis of the irradiated group (IR) increased compared to the control group, and the expression was especially noticeable at a site where collagen fibers are increased and thickened. On the other hand, it was found that vimentin expression decreased in the darapladib-treated group (IR/Dara), and in particular, the expression was reduced in the high-concentration treated group (40 mg/kg) similarly to the control group (FIG. 8).

Having described in detail a specific part of the present disclosure above, it is clear for those skilled in the art that this specific description is merely preferred example embodiments, and the scope of the present disclosure is not limited thereby. Thus, the substantial scope of the present disclosure is defined by the appended claims and their equivalents.

## Claims

1. A pharmaceutical composition for preventing or treating a skin disease caused by radiation irradiation, comprising darapladib or a pharmaceutically acceptable salt thereof as an active ingredient.

2. The pharmaceutical composition of claim 1, wherein the darapladib or pharmaceutically acceptable salt thereof inhibits expression of one or more selected from the group consisting of collagen, α-SMA, and vimentin.

3. The pharmaceutical composition of claim 1, wherein the darapladib or pharmaceutically acceptable salt thereof suppresses differentiation from fibroblasts to myofibroblasts; or overgrowth of fibroblasts.

4. The pharmaceutical composition of claim 1, wherein the skin disease comprises fibrosis or dermatitis.

5. The pharmaceutical composition of claim 1, wherein the radiation irradiation is high-dose irradiation.

6. The pharmaceutical composition of claim 5, wherein the radiation ranges from 10 to 40 Gy.

7. The pharmaceutical composition of claim 1, wherein the pharmaceutical composition has formulation in the form of one or more external skin agents selected from the group consisting of injectable formulations such as aqueous solutions, suspensions, and emulsions, pills, capsules, granules, tablets, creams, gels, patches, sprays, ointments, plasters, lotions, liniments, pastas, and cataplasmas.

8. A health functional food composition for preventing or ameliorating a skin disease caused by radiation irradiation, comprising darapladib or a foodologically acceptable salt thereof as an active ingredient.

9. A cosmetic composition for preventing or ameliorating a skin disease caused by radiation irradiation, comprising darapladib or a cosmetically acceptable salt thereof as an active ingredient.

10. The cosmetic composition of claim 9, wherein the cosmetic composition has one or more formulations selected from the group consisting of skin lotions, skin softeners, skin toners, astringents, milk lotions, moisture lotions, nourishing lotions, massage creams, nourishing creams, moisture creams, hand creams, foundations, essences, nourishing essences, packs, soaps, cleansing foams, cleansing lotions, cleansing creams, body lotions, and body cleansers.
